# EUROPEAN PATENT APPLICATION

(11) **EP 4 349 805 A1**
(43) Date of publication of application: **10.04.2024**
(21) Application number: 22815872.1
(22) Date of filing: 19.05.2022
(51) Int. Cl.: C07C 17/16, C07C 17/263, C07C 17/361, C07C 19/08, C07C 21/18

(54) **METHOD FOR PRODUCING FLUOROHYDROCARBON**

(30) Priority: 31.05.2021 JP 2021091662
(71) Applicant: Zeon Corporation, Tokyo 100-8246 (JP); Kyushu University, National University Corporation, Nishi-ku Fukuoka-shi Fukuoka 819-0395 (JP)
(72) Inventor: MATSUURA Go, Tokyo 100-8246 (JP); INADA Keita, Tokyo 100-8246 (JP); WATANABE Takayuki, Fukuoka-shi, Fukuoka 819-0395 (JP); TANAKA Manabu, Fukuoka-shi, Fukuoka 819-0395 (JP); MATSUI Kazuki, Fukuoka-shi, Fukuoka 819-0395 (JP)
(74) Representative: Maiwald GmbH
(86) International application number: PCT/JP2022/020867
(87) International publication number: WO 2022/255120

(57) **Abstract**

Provided is a production method that enables simple and efficient production of a fluorinated hydrocarbon by a gas phase flow method without using a catalyst. The method of producing a fluorinated hydrocarbon includes continuously supplying a gaseous fluorine-containing inorganic compound to an electrical discharge zone in a plasma apparatus and subsequently causing migration to outside of the electrical discharge zone in the plasma apparatus, continuously supplying a feedstock gas containing an inert gas and a compound represented by formula 1: CH₃-R (R is a hydrogen atom, a chlorine atom, a bromine atom, an iodine atom, or an organic group other than a hydrocarbon group) to outside of the electrical discharge zone in the plasma apparatus, and subsequently continuously releasing, to outside of the plasma apparatus, gas that is contained outside of the electrical discharge zone in the plasma apparatus.

## Description

### TECHNICAL FIELD

The present disclosure relates to a method of producing a fluorinated hydrocarbon.

### BACKGROUND

Monochloromethane and other such fluorinated hydrocarbons are widely used in applications such as for etching gases in microfabrication of semiconductors.

In a known method of producing monofluoromethane, methyl chloride (CH₃Cl) and hydrogen fluoride are reacted in a gas phase in the presence of a fluorination catalyst to obtain a mixed gas containing monofluoromethane, and then monofluoromethane is separated and purified from this mixed gas.

### CITATION LIST

### Patent Literature

PTL 1: JP2006-111611A

### SUMMARY

### (Technical Problem)

However, in the method of producing monofluoromethane described above, production of a fluorination catalyst has been a significant burden, and continuous production has been difficult due to reduction of yield accompanying reduction of catalyst activity, for example.

An object of the present disclosure is to provide a production method that enables simple and efficient production of monofluoromethane and other such fluorinated hydrocarbons by a gas phase flow method without using a catalyst.

The present disclosure aims to advantageously solve the problems set forth above and primary features thereof are as follows.
[1] A method of producing a fluorinated hydrocarbon comprising: continuously supplying a gaseous fluorine-containing inorganic compound to an electrical discharge zone in a plasma apparatus and subsequently causing migration to outside of the electrical discharge zone in the plasma apparatus,
   continuously supplying a feedstock gas containing an inert gas and a compound represented by formula 1: CH₃-R, where R is a hydrogen atom, a chlorine atom, a bromine atom, an iodine atom, or an organic group other than a hydrocarbon group, to outside of the electrical discharge zone in the plasma apparatus, and
   subsequently continuously releasing, to outside of the plasma apparatus, gas that is contained outside of the electrical discharge zone in the plasma apparatus.
[2] The method of producing a fluorinated hydrocarbon according to the foregoing [1], wherein the plasma apparatus is an apparatus that forms an atmospheric pressure thermal plasma.
[3] The method of producing a fluorinated hydrocarbon according to the foregoing [2], wherein the atmospheric pressure thermal plasma is formed through arc discharge or high-frequency discharge.
[4] The method of producing a fluorinated hydrocarbon according to any one of the foregoing [1] to [3], wherein the gaseous fluorine-containing inorganic compound is one or more selected from the group consisting of SF₄, SF₆, SOF₂, SO₂F₂, HF, NF₃, CF₄, COF₂, BF₃, and SiF₄.
[5] The method of producing a fluorinated hydrocarbon according to any one of the foregoing [1] to [4], wherein the inert gas is one or more selected from the group consisting of N₂ and Ar.
[6] The method of producing a fluorinated hydrocarbon according to any one of the foregoing [1] to [5], wherein proportional content of the compound represented by formula 1 among the feedstock gas is not less than 1 volume% and not more than 50 volume%.
[7] The method of producing a fluorinated hydrocarbon according to any one of the foregoing [1] to [6], wherein a volume ratio of the gaseous fluorine-containing inorganic compound relative to the compound represented by formula 1 is 0.8 or more.
[8] The method of producing a fluorinated hydrocarbon according to the foregoing [7], wherein the volume ratio is 1.8 or more.
[9] The method of producing a fluorinated hydrocarbon according to any one of the foregoing [1] to [8], wherein the fluorinated hydrocarbon is monofluoromethane.

Herein, "electrical discharge zone in a plasma apparatus" refers to a space inside of a plasma apparatus where an electric field causes breakdown of electrical insulation of a gas and where electrical current flows.

"Gaseous fluorine-containing inorganic compound" refers to a fluorine-containing inorganic compound that is a gas in a standard state (298 K, atmospheric pressure). "Inorganic compound" refers to a compound that does not include a carbon atom or a compound that includes one carbon atom and does not include a hydrogen atom.

"Fluorinated hydrocarbon" refers to a compound in which at least one hydrogen atom of a hydrocarbon compound has been replaced by a fluorine atom and is inclusive of a compound in which all of the hydrogen atoms of a hydrocarbon compound have been replaced by fluorine atoms.

A fluorinated hydrocarbon is presumed to be produced as follows according to the presently disclosed method of producing a fluorinated hydrocarbon.

When the gaseous fluorine-containing inorganic compound is supplied to the electrical discharge zone in the plasma apparatus, at least one of a fluorine ion and a fluorine radical (hereinafter, referred to as a "fluorine source") is generated in the electrical discharge zone. When this fluorine source is supplied to outside of the electrical discharge zone in the plasma apparatus, the compound represented by formula 1: CH₃-R (R is a hydrogen atom, a chlorine atom, a bromine atom, an iodine atom, or an organic group other than a hydrocarbon group) is also supplied to outside of the electrical discharge zone. Various active species are generated from the compound of formula 1 outside of the electrical discharge zone, but decomposition of the compound of formula 1 is inhibited to a suitable degree, and the generated active species include a sufficient amount of radicals or ions such as CH₃ radicals or CH₃ ions that include a partial structure of the compound of formula 1 (hereinafter, these radicals and ions are also referred to as "CH₃ radicals or the like").

Moreover, when the fluorine source and the active species (particularly CH₃ radicals and CH₃ ions) that are contained outside of the electrical discharge zone are continuously released to outside of the plasma apparatus, the fluorine source and the active species (particularly CH₃ radicals and CH₃ ions) bond to produce a fluorinated hydrocarbon. By utilizing a plasma in this manner, it is possible to simply and efficiently produce a fluorinated hydrocarbon in a gas phase without using a catalyst.

In the presently disclosed method of producing a fluorinated hydrocarbon, an apparatus that forms an atmospheric pressure thermal plasma can be used as the plasma apparatus, and a reaction field created by this atmospheric pressure thermal plasma can be used. This is advantageous because it enables higher throughput per unit time using a compact apparatus. The atmospheric pressure thermal plasma can be formed by arc discharge or high-frequency discharge.

The electrical discharge zone of the atmospheric pressure thermal plasma is a zone where an electrical field causes breakdown of electrical insulation of a gas and where electrical current flows. In the case of a plasma apparatus that generates a plasma using electrodes, the electrical discharge zone is a space between opposing electrodes, whereas in the case of a method in which a flame is drawn out from between electrodes such as with a plasma jet, the electrical discharge zone corresponds to the flame zone thereof, and in the case of a plasma apparatus that generates a plasma by a means other than electrodes, the electrical discharge zone is a space corresponding thereto. The plasma apparatus that generates a plasma by a means other than electrodes may be an inductively coupled plasma (ICP) apparatus or the like. The electrical discharge zone of the atmospheric pressure thermal plasma normally includes a high temperature section that reaches 10,000 K or higher.

In proximity to the electrical discharge zone of the atmospheric pressure thermal plasma, a temperature of 1,000 K to 3,000 K is reached through radiation of the plasma, and excitation and dissociation reactions of chemical species can occur. For example, in the case of a plasma apparatus that generates a plasma using electrodes, a 1,000 K to 3,000 K temperature zone is included in proximity to an anode at a downstream side. This temperature zone constitutes outside of the electrical discharge zone of the atmospheric pressure thermal plasma.

In the presently disclosed method of producing a fluorinated hydrocarbon, the gaseous fluorine-containing inorganic compound is preferably one or more selected from the group consisting of SF₄, SF₆, SOF₂, SO₂F₂, HF, NF₃, CF₄, COF₂, BF₃, and SiF₄. These compounds are advantageous because they enable simple generation of a fluorine source in the electrical discharge zone.

Moreover, in the presently disclosed method of producing a fluorinated hydrocarbon, the inert gas can be one or more selected from the group consisting of N₂ and Ar.

Furthermore, in the presently disclosed method of producing a fluorinated hydrocarbon, the proportional content of the compound of formula 1 among the feedstock gas is preferably not less than 1 volume% and not more than 85 volume%. When the proportional content is within the range set forth above, this enables efficient production of a fluorinated hydrocarbon that is a target substance.

Also, in the presently disclosed method of producing a fluorinated hydrocarbon, a volume ratio of the gaseous fluorine-containing inorganic compound relative to the compound of formula 1 is preferably 0.8 or more. When the volume ratio is not less than the lower limit set forth above, this enables efficient production of a fluorinated hydrocarbon that is a target substance.

Moreover, in the presently disclosed method of producing a fluorinated hydrocarbon, the volume ratio of the gaseous fluorine-containing inorganic compound relative to the compound of formula 1 is preferably 1.8 or more.

In the presently disclosed method of producing a fluorinated hydrocarbon, monofluoromethane can be set as a target substance.

### (Advantageous Effect)

According to the present disclosure, it is possible to provide a production method that enables simple and efficient production of a fluorinated hydrocarbon by a gas phase flow method without using a catalyst. The presently disclosed production method is advantageous because it makes it possible to avoid reduction of yield caused by reduction of catalyst activity and enables continuous production of a fluorinated hydrocarbon.

### BRIEF DESCRIPTION OF THE DRAWINGS

In the accompanying drawings:

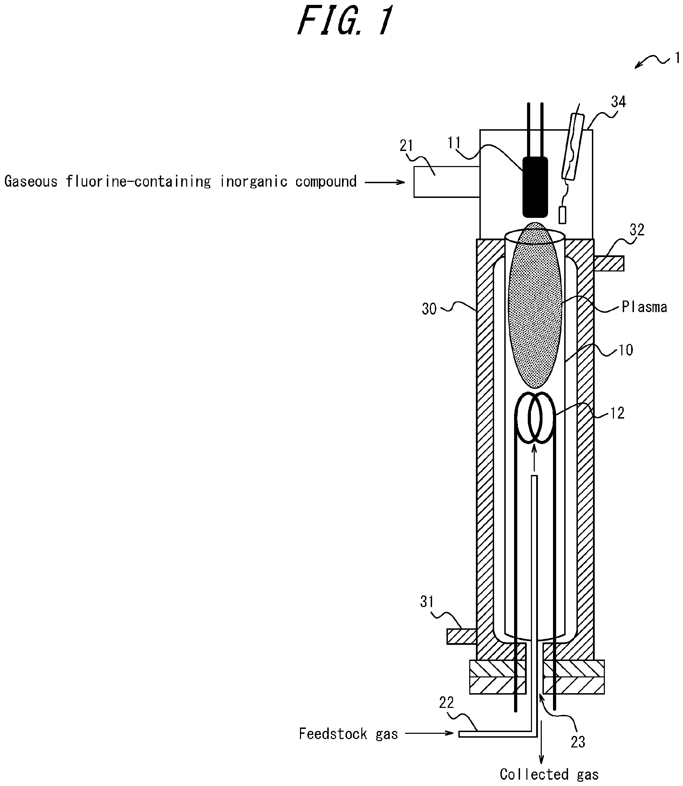

FIG. 1 illustrates one example of a plasma apparatus that can be used in the presently disclosed method of producing a fluorinated hydrocarbon.

### DETAILED DESCRIPTION

The following provides a detailed description of embodiments of the present disclosure.

### [Gaseous fluorine-containing inorganic compound]

The gaseous fluorine-containing inorganic compound may be any gaseous inorganic compound that includes at least one fluorine atom. The number of fluorine atoms is normally 8 or less.

The gaseous fluorine-containing inorganic compound may be SF₄, SF₆, SOF₂, SO₂F₂, HF, NF₃, CF₄, COF₂, BF₃, SiF₄, or the like. In terms of ease of handling, SF₆, NF₃, CF₄, BF₃, and SiF₄ are preferable. The gaseous fluorine-containing inorganic compound may be just one compound or may be any combination of two or more compounds.

### [Compound of formula 1]

The compound of formula 1 is a compound that is represented by formula 1: CH₃-R (R is a hydrogen atom, a chlorine atom, a bromine atom, an iodine atom, or an organic group other than a hydrocarbon group). The compound of formula 1 may be just one compound or may be two or more compounds used together.

The "organic group other than a hydrocarbon group" is a functional group that includes at least one carbon atom (excluding functional groups formed of only carbon atoms and hydrogen atoms) or a functional group that includes one or more selected from oxygen, nitrogen, and sulfur and does not include a carbon atom. The organic group other than a hydrocarbon group may be an oxygen-containing organic group, a nitrogen-containing organic group, or a sulfur-containing organic group.

The oxygen-containing organic group may be a hydroxy (-OH), carboxy (-COOH), formyl (-CHO), formyloxy (-O-CH(=O)), acyl (-CR¹(=O)), acyloxy (-O-CR¹(=O)), alkoxy (-OR¹), alkoxycarbonyl (-C(=O)-OR¹), or the like, where R¹ is an alkyl, preferably a C1 to C4 alkyl, and more preferably a methyl or ethyl.

The nitrogen-containing organic group may be an unsubstituted amino (-NH₂), substituted amino (-NR²R³), nitro (-NO₂), cyano (-CN), or the like, where R² and R³ are independently a hydrogen or alkyl, but at least one of R² and R³ is an alkyl, and the alkyl is preferably a C1 to C4 alkyl, and more preferably a methyl or ethyl.

The sulfur-containing organic group may be a mercapto (-SH), sulfo (-SO₃H), alkylthio (-SR⁴), or the like, where R⁴ is an alkyl, preferably a C1 to C4 alkyl, and more preferably a methyl.

R is preferably a hydrogen atom, chlorine atom, bromine atom, iodine atom, hydroxy (-OH), alkoxy (-OR¹), acyl (-CR¹(=O)), or substituted amino (-NR²R³) (R¹, R², and R³ are as previously described), and is more preferably a hydrogen atom, chlorine atom, hydroxy, methoxy, acetyl, or dimethylamino.

The compound of formula 1 may be CH₄, CH₃OH, CH₃Cl, CH₃Br, CH₃I, CH₃CHO, HCOOCH₃, CH₃COOCH₃, CH₃COOC₂H₅, CH₃NH₂, (CH₃)₂NH, (CH₃)₃N, CH₃CN, CH₃NO₂, CH₃SH, CH₃SCH₃, CH₃OCH₃, CH₃OC₂H₅, CH₃COCH₃, CH₃COC₂H₅, or the like, and, in terms of ease of handling, is preferably CH₄, CH₃OH, CH₃Cl, CH₃COCH₃, CH₃OCH₃, or (CH₃)₃N, and more preferably CH₃OH.

### [Inert gas]

The inert gas may be N₂, He, Ne, Ar, Xe, Kr, CO, CO₂, or the like, is preferably N₂, Ar, He, CO, or CO₂, and is more preferably N₂ or Ar. The inert gas may be just one type of gas or may be two or more types of gases used together.

### [Feedstock gas]

The feedstock gas contains the compound of formula 1 and the inert gas. The compound of formula 1 may be a gas, a liquid, or a solid in a standard state (atmospheric pressure, 298 K), but is a gas when the feedstock gas is introduced to outside of the electrical discharge zone in the plasma apparatus.

The proportional contents of the compound of formula 1 and the inert gas in the feedstock gas can be adjusted to any proportions without any specific limitations. The proportional content of the compound of formula 1 among the feedstock gas is preferably 1 volume% or more, and more preferably 5 volume% or more, and is preferably 85 volume% or less, and more preferably 80 volume% or less. A remaining portion of the feedstock gas other than the compound of formula 1 and the inert gas is preferably impurities that are unavoidably mixed in from the surrounding environment.

The feedstock gas contains the compound of formula 1 and the inert gas at outside of the electrical discharge zone in the plasma apparatus. The compound of formula 1 and the inert gas may each be separately supplied as a gas to outside of the electrical discharge zone in the plasma apparatus so as to provide the feedstock gas thereat, all of the compound of formula 1 and the inert gas may be premixed to obtain a gas, and this gas may be supplied to outside of the electrical discharge zone in the plasma apparatus so as to provide the feedstock gas thereat, or some of the compound of formula 1 and the inert gas may be premixed to obtain a gas, and this gas and a gas of the remainder of the compound of formula 1 and the inert gas may be separately supplied to outside of the electrical discharge zone in the plasma apparatus so as to provide the feedstock gas thereat.

In a case in which the compound of formula 1 is a gas or a liquid having a sufficiently high vapor pressure that can easily be vaporized by heating or the like in a standard state, the compound of formula 1 can be supplied to outside of the electrical discharge zone in the plasma apparatus as a gas without providing a separate vaporization chamber or the like. Control of the supply flow rate can be performed using a mass flow controller or the like.

In a case in which the compound of formula 1 is a liquid having a low vapor pressure or is a solid in a standard state, the compound of formula 1 can be vaporized in a separately provided vaporization chamber and then be supplied to a gas phase flow reactor. In the case of a solid, the solid can be introduced into the vaporization chamber after being converted to a liquid by heating.

For example, the compound of formula 1 can be vaporized by introducing the compound of formula 1, in a liquid state, into a vaporization chamber held at a temperature and pressure at which sufficient vaporization of the compound of formula 1 occurs. The temperature and pressure of the vaporization chamber are preferably held at a temperature and pressure that enable instantaneous vaporization of the compound of formula 1. The use of such a vaporization chamber makes it possible to continuously introduce the compound of formula 1 into the vaporization chamber as a liquid, cause instantaneous vaporization thereof in the vaporization chamber, and then continuously supply the compound of formula 1 to outside of the electrical discharge zone in the plasma apparatus as a gas. Control of the supply flow rate can be performed by using a mass flow controller or the like to control gas that has been vaporized in the vaporization chamber or can be performed by using a liquid mass flow controller or the like to control continuous introduction of the compound of formula 1 into the vaporization chamber in a liquid state. When the compound of formula 1 that has been vaporized is introduced to outside of the electrical discharge zone in the plasma apparatus, the compound of formula 1 may be diluted with the inert gas.

### [Electrical discharge zone]

A reaction field that is created by an atmospheric pressure thermal plasma can be used in the presently disclosed production method. More specifically, using a plasma apparatus that forms an atmospheric pressure thermal plasma, a fluorine source that is generated in an electrical discharge zone of the atmospheric pressure thermal plasma can be supplied to outside of the electrical discharge zone of the atmospheric pressure thermal plasma, and this outside zone can be used as a reaction field.

In the presently disclosed production method, at least one of a fluorine ion and a fluorine radical can be generated as a fluorine source from the gaseous fluorine-containing inorganic compound in the electrical discharge zone in the plasma apparatus, and then this fluorine source can be caused to migrate to outside of the electrical discharge zone.

Formation of the atmospheric pressure thermal plasma can be performed using an electrical method. For example, arc discharge, high-frequency discharge, pulse discharge, or multiphase alternating current discharge can be used to form the atmospheric pressure thermal plasma. The arc discharge may a direct current arc or may be an alternating current arc. A multiphase alternating current arc is preferable in terms of enabling processing with a high flow rate. In the case of high-frequency discharge, an inductively coupled high-frequency discharge plasma is advantageous in terms of efficient processing.

It is preferable that the gaseous fluorine-containing inorganic compound is caused to continuously flow and generate the fluorine source in the electrical discharge zone. The space velocity of the gaseous fluorine-containing inorganic compound when it is caused to continuously flow is not specifically limited but is preferably 0.01 h⁻¹ or more, more preferably 0.1 h⁻¹ or more, and even more preferably 0.3 h⁻¹ or more, and is preferably 100,000 h⁻¹ or less, more preferably 50,000 h⁻¹ or less, and even more preferably 10,000 h⁻¹ or less. A space velocity that is within any of the ranges set forth above enables sufficient supply of the fluorine source and efficient production of a fluorinated hydrocarbon.

In the presently disclosed production method, the feedstock gas is introduced to outside of the electrical discharge zone in the plasma apparatus. For example, the feedstock gas can be introduced to outside of an electrical discharge zone of an atmospheric pressure thermal plasma. By introducing the feedstock gas to outside of the electrical discharge zone, it is possible to generate a sufficient amount of CH₃ radicals through the compound of formula 1 in the feedstock gas and to efficiently produce a fluorinated hydrocarbon. In contrast, decomposition of the compound of formula 1 proceeds and it is difficult to ensure a sufficient amount of CH₃ radicals or the like in a situation in which the feedstock gas is introduced into the electrical discharge zone.

The space velocity of the feedstock gas when it is caused to continuously flow outside of the electrical discharge zone is not specifically limited but is preferably 0.01 h⁻¹ or more, more preferably 0.1 h⁻¹ or more, and even more preferably 0.3 h⁻¹ or more, and is preferably 100,000 h⁻¹ or less, more preferably 50,000 h⁻¹ or less, and even more preferably 10,000 h⁻¹ or less. A space velocity that is within any of the ranges set forth above makes it possible to avoid complication of plasma formation and enables efficient production of a fluorinated hydrocarbon.

In the presently disclosed production method, the volume ratio of the gaseous fluorine-containing inorganic compound relative to the compound of formula 1 in the feedstock gas can be adjusted to any ratio without any specific limitations. The volume ratio of the fluorine-containing inorganic compound relative to the compound of formula 1 is preferably 0.8 or more in terms of inhibiting production of hydrocarbon by-products, and is more preferably 1.8 or more for production of the target substance. The volume ratio can be 100 or less, and may be set as 25 or less, for example.

### [Fluorinated hydrocarbon]

A fluorinated hydrocarbon can be obtained by continuously releasing, to outside of the plasma apparatus, gas that is contained outside of the electrical discharge zone in the plasma apparatus. The gas that is contained outside of the electrical discharge zone in the plasma apparatus contains the fluorine source and active species such as CH₃ radicals or the like that have been generated from the compound of formula 1. By continuously releasing these to outside of the plasma apparatus, the fluorine source and the active species such as CH₃ radicals or the like (particularly CH₃ radicals and CH₃ ions) bond to produce a fluorinated hydrocarbon. The continuous release can be performed with a space velocity corresponding to the continuous flow of the gaseous fluorine-containing inorganic compound and the feedstock gas.

After the gas has been released to outside of the plasma apparatus, the gas may be further introduced into a heat exchanger and may be cooled. The mechanism of the heat exchanger is not specifically limited and may be air cooling, water cooling, or the like. Since the released material may contain hydrocarbons and the like other than the fluorinated hydrocarbon that is the target substance, the released material may be subjected to a separation and purification step. Examples of separation and purification methods that can be used include distillation, absorption by a solution or the like, and membrane separation.

The presently disclosed production method enables production of a fluorinated hydrocarbon and is advantageous in terms of producing a fluorinated hydrocarbon having a carbon number of 1 or 2. Specifically, the fluorinated hydrocarbon may be CH₃F, CH₂F₂, CHF₃, CF₄, C₂H₂F₂, or the like. Examples of applications for monofluoromethane (CH₃F) include as an etching gas, whereas examples of applications for difluoromethane (CH₂F₂) and trifluoromethane (CHF₃) include as a chlorofluorocarbon alternative material or etching gas.

In production of monofluoromethane, a combination in which the gaseous fluorine-containing inorganic compound is SF₆ and the compound of formula 1 is CH₃OH is preferable.

### [Plasma apparatus]

FIG. 1 illustrates one example of a plasma apparatus that can be used in the presently disclosed production method. This plasma apparatus is an apparatus that uses arc discharge.

The plasma apparatus 1 includes a combustion tube 10 inside of a cooling jacket 30. The cooling jacket 30 has a structure in which cooling water flows. The combustion tube 10 is preferably made of a ceramic in terms of heat resistance.

A cathode 11 is installed above the combustion tube 10, and an anode 12 is installed inside of the combustion tube 10. An ignition wire 34 is arranged at the cathode side. Voltage is applied between the cathode 11 and the anode 12 to cause electrical discharge. Conditions of the electrical discharge are not specifically limited and can be set as a voltage of 300 V to 600 V and a current of 1 A to 20 A, for example. The size of the combustion tube 10 and the interelectrode distance can be set as appropriate. For a plasma that is generated by the electrical discharge, a side corresponding to the anode 12 is taken to be downstream of the plasma.

In the plasma apparatus 1, a first gas supply tube 21 is connected at a side corresponding to the cathode 11. A gaseous fluorine-containing inorganic compound 21 is supplied from this supply tube 21. By supplying the gaseous fluorine-containing inorganic compound from this position, the gaseous fluorine-containing inorganic compound is converted to a plasma state in a plasma flame section and can generate a fluorine source. The generated fluorine source migrates to a downstream side of the plasma and is supplied to outside of the electrical discharge zone at a lower section (opposite side to the cathode) of the anode 12 in the plasma apparatus 1. The lower section (opposite side to the cathode) of the anode 12 includes a 1,000 K to 3,000 K temperature zone.

A bottom section of the plasma apparatus 1 is provided with a gas collection port 23. A second gas supply tube 22 is inserted into the gas collection port 23. A feedstock gas is supplied from this supply tube 22. By releasing the feedstock gas toward a side corresponding to the cathode 11, the feedstock gas is caused to reach a downstream side of the plasma and to generate active species such as CH₃ radicals or the like at outside of the electrical discharge zone. Through adjustment of the length of the gas supply tube 22 in the combustion tube 10, it is possible to adjust the supply position of the feedstock gas, to control decomposition of the compound of formula 1, and to cause generation of a sufficient amount of CH₃ radicals or the like.

Gas at a downstream side of the plasma contains the fluorine source and active species such as CH₃ radicals or the like that have been generated from the compound of formula 1, and this gas can be collected outside of the plasma apparatus 1 from the gas collection port 23. During this collection, the fluorine source and active species such as CH₃ radicals or the like (particularly CH₃ radicals and CH₃ ions) bond to produce a fluorinated hydrocarbon. The collection can be performed by connecting a vacuum pump to the gas collection port 23, for example.

### EXAMPLES

The following provides a more detailed description of the present disclosure through examples. However, the present disclosure is not limited by these examples. In the examples, a plasma apparatus corresponding to FIG. 1 was used.

### (Example 1)

A long DC arc plasma apparatus made of Hastelloy (volume: 6.5 L; interelectrode distance: 300 mm) was used as the plasma apparatus 1. A cylindrical ceramic tube made of mullite (internal diameter: 42 mm; length: 600 mm) was installed as the combustion tube 10 inside of the plasma apparatus. N₂ serving as a plasma mother gas was introduced into the ceramic tube from the first gas supply tube 21 at 26.6 slm, and plasma ignition was performed with a current value of 10 A.

After plasma ignition, 26.6 slm of N₂ and 0.10 slm of SF₆ were introduced from the first gas supply tube 21 such as to flow in an electrical discharge zone of the plasma, and then CH₃OH was bubbled with 3.4 slm of N₂, and CH₃OH equivalent to 0.50 slm was introduced, together with this N₂, through the second gas supply tube. Gas at a downstream side of the plasma was discharged outside of the system from the gas collection port 23. Collected gas that had been discharged outside of the system was detoxified using KOH aqueous solution and was then trapped in an aluminum bag.

The trapped gas was analyzed by gas chromatography-mass spectrometry (GC-MS) (Agilent 7890A produced by Agilent Technologies, Inc.) and flame ionization detection gas chromatography (GC-FID) (Agilent 6890N produced by Agilent Technologies, Inc.). The yield of a fluorinated hydrocarbon was determined from area values for components in GC-MS and GC-FID that were obtained through analysis. The results are shown in Table 1.

### (Examples 2 to 6)

Examples 2 to 6 are the same as Example 1 with the exception that the flow rate of SF₆ was changed to an amount indicated in Table 1. The results are shown in Table 1.

### (Examples 7 to 9)

Examples 7 to 9 are the same as Example 1 with the exception that the flow rate of SF₆ was changed to an amount indicated in Table 1, CH₃OH was changed to CH₄ and was set as an amount indicated in Table 1, and N₂ was an entrained gas of CH₄ rather than being bubbled. The results are shown in Table 1.

### (Examples 10 to 12)

Examples 10 to 12 are the same as Example 1 with the exception that SF₆ was changed to CF₄ and was set as an amount indicated in Table 1. The results are shown in Table 1.

### (Examples 13 to 15)

Examples 13 to 15 are the same as Example 1 with the exception that SF₆ was set as an amount indicated in Table 1, CH₃OH was changed to CH₄, and N₂ was entrained gas of CH₄ rather than being bubbled. The results are shown in Table 1.

### (Examples 16 to 18)

Examples 16 to 18 are the same as Example 1 with the exception that SF₆ was set as an amount indicated in Table 1, and CH₃OH was changed to CH₃COCH₃. The results are shown in Table 1.

### (Examples 19 to 21)

Examples 19 to 21 are the same as Example 1 with the exception that SF₆ was set as an amount indicated in Table 1, and CH₃OH was changed to CH₃COOCH₃. The results are shown in Table 1.

**[Table 1]**

| Example | Mother gas | Flow rate [slm] | Gaseous fluorine-containing inorganic compound | Flow rate of inorganic compound [slm] | Compound of formula 1 | Flow rate of compound of formula 1 [slm] | Bubbled or entrained gas | Flow rate [sim] | CH₃F yield | C₂H₂F₂ yield |
|---|---|---|---|---|---|---|---|---|---|---|
| Example 1 | | | SF₆ | 0.10 | CH₃OH | | | | 0.3% | - |
| Example 2 | | | | 0.25 | | | | | 0.8% | - |
| Example 3 | | | | 0.50 | | | | | 1.8% | - |
| Example 4 | | | | 1.00 | | | | | 2.8% | - |
| Example 5 | | | | 2.50 | | | | | 5.6% | - |
| Example 6 | | | | 5.00 | | | | | 6.6% | - |
| Example 7 | | | | 1.00 | CH₄ | | | | - | 0.3% |
| Example 8 | | | | 2.50 | | | | | - | 0.7% |
| Example 9 | | | | 5.00 | | | | | - | 1.0% |
| Example 10 | | | CF₄ | 1.00 | CH₃OH | | | | - | 0.1% |
| Example 11 | N₂ | 26.6 | | 2.50 | | 0.50 | N₂ | 3.4 | - | 0.3% |
| Example 12 | | | | 5.00 | | | | | - | 0.6% |
| Example 13 | | | SF₆ | 1.00 | CH₄ | | | | - | 0.1% |
| Example 14 | | | | 2.50 | | | | | - | 0.3% |
| Example 15 | | | | 5.00 | | | | | - | 0.7% |
| Example 16 | | | | 1.00 | CH₃COCH₃ | | | | - | 0.3% |
| Example 17 | | | | 1.50 | | | | | - | 0.3% |
| Example 18 | | | | 2.50 | | | | | - | 0.4% |
| Example 19 | | | | 1.10 | CH₃COOCH₃ | | | | - | 0.5% |
| Example 20 | | | | 1.70 | | | | | - | 0.9% |
| Example 21 | | | | 2.70 | | | | | - | 0.9% |

| | | | | | | | | | | |
|---|---|---|---|---|---|---|---|---|---|---|
| * A dash (-) for yield indicates below the limit of detection. | | | | | | | | | | |

It can be seen from Table 1 that a fluorinated hydrocarbon could be produced without using a catalyst in the examples.

### INDUSTRIAL APPLICABILITY

According to the present disclosure, it is possible to simply and efficiently produce a fluorinated hydrocarbon by a gas phase flow method without using a catalyst. The presently disclosed production method makes it possible to avoid reduction of yield caused by reduction of catalyst activity, enables continuous production of a fluorinated hydrocarbon that is useful in applications such as for an etching gas, and has high industrial applicability.

### REFERENCE SIGNS LIST

- 1: plasma apparatus
- 10: combustion tube
- 11: cathode
- 12: anode
- 21: first gas supply tube
- 22: second gas supply tube
- 23: gas collection port
- 30: cooling jacket
- 31: cooling water supply port
- 32: cooling water discharge port
- 34: ignition wire

## Claims

1. A method of producing a fluorinated hydrocarbon comprising:
continuously supplying a gaseous fluorine-containing inorganic compound to an electrical discharge zone in a plasma apparatus and subsequently causing migration to outside of the electrical discharge zone in the plasma apparatus,
continuously supplying a feedstock gas containing an inert gas and a compound represented by formula 1: CH₃-R, where R is a hydrogen atom, a chlorine atom, a bromine atom, an iodine atom, or an organic group other than a hydrocarbon group, to outside of the electrical discharge zone in the plasma apparatus, and
subsequently continuously releasing, to outside of the plasma apparatus, gas that is contained outside of the electrical discharge zone in the plasma apparatus.

2. The method of producing a fluorinated hydrocarbon according to claim 1, wherein the plasma apparatus is an apparatus that forms an atmospheric pressure thermal plasma.

3. The method of producing a fluorinated hydrocarbon according to claim 2, wherein the atmospheric pressure thermal plasma is formed through arc discharge or high-frequency discharge.

4. The method of producing a fluorinated hydrocarbon according to claim 1, wherein the gaseous fluorine-containing inorganic compound is one or more selected from the group consisting of SF₄, SF₆, SOF₂, SO₂F₂, HF, NF₃, CF₄, COF₂, BF₃, and SiF₄.

5. The method of producing a fluorinated hydrocarbon according to claim 1, wherein the inert gas is one or more selected from the group consisting of N₂ and Ar.

6. The method of producing a fluorinated hydrocarbon according to claim 1, wherein proportional content of the compound represented by formula 1 among the feedstock gas is not less than 1 volume% and not more than 50 volume%.

7. The method of producing a fluorinated hydrocarbon according to claim 1, wherein a volume ratio of the gaseous fluorine-containing inorganic compound relative to the compound represented by formula 1 is 0.8 or more.

8. The method of producing a fluorinated hydrocarbon according to claim 7, wherein the volume ratio is 1.8 or more.

9. The method of producing a fluorinated hydrocarbon according to any one of claims 1 to 8, wherein the fluorinated hydrocarbon is monofluoromethane.
